# EUROPÄISCHE PATENTSCHRIFT

(11) **EP 3 064 251 B1**
(45) Veröffentlichungstag und Bekanntmachung des Hinweises auf die Patenterteilung: **01.01.2020**
(21) Anmeldenummer: 15158066.9
(22) Anmeldetag: 06.03.2015
(51) Int. Cl.: A61M 37/00, A61D 7/00, A61B 5/15, A61B 5/151, A61B 17/20

(54) **Hautstechwerkzeug zum lokalen Aufstechen einer menschlichen oder einer tierischen Haut und Handgerät**
Skin puncturing tool for local puncturing of human or animal skin and hand-held device
Auto-piqueur destiné au perçage local d'une peau humaine ou animale et appareil manuel

(43) Veröffentlichungstag der Anmeldung: 07.09.2016
(73) Patentinhaber: MT.DERM GmbH, 14167 Berlin (DE)
(72) Erfinder: Scherkowski, Dirk, 12489 Berlin (DE)
(74) Vertreter: Bittner, Thomas L.

(56) Entgegenhaltungen:
- EP-A1- 1 576 982
- US-A1- 2005 065 483
- US-A1- 2007 038 181
- US-A1- 2012 029 549

## Beschreibung

Die Erfindung betrifft ein Hautstechwerkzeug zum lokalen Aufstechen einer menschlichen oder einer tierischen Haut sowie ein Handgerät.

### Hintergrund

Hautstechwerkzeuge werden in Handgeräten zum lokalen Aufstechen einer menschlichen oder einer tierischen Haut verwendet. Solche Handgeräte können zum Einbringen einer Substanz über die Haut dienen, zum Beispiel eines Farbstoffs in Verbindung mit permanentem Makeup oder der Ausbildung eines Tattoos. Aber auch medizinische oder kosmetische Stoffe können unter Verwendung eines solchen Handgeräts in die Haut oder durch die Haut appliziert werden.

Hautstechwerkzeuge sind in Form von Kanülen oder Vollmaterialnadeln bekannt.

Als Handgeräte kommen Vorrichtungen zum Einsatz, die über eine Antriebseinrichtung verfügen, welche repetierend eine Antriebskraft zum Aus- und Einfahren des Hautstechwerkzeugs zur Verfügung stellt. Hierbei kann das Hautstechwerkzeug mit einer Einzelnadel oder in Form von einer Nadelgruppe gebildet sein. Darüber hinaus sind Nadelplatten zum Aufstechen der Haut bekannt.

Aus dem Dokument US 2005/065483 A1 ist ein Hautstechwerkzeug bekannt, welches mehrere Hohlnadeln aufweist, die sich flexibel krümmen können, wenn die jeweilige Spitze der Hohlnadeln beim Einstechen mit Druck beaufschlagt wird. In einer Ausführungsform weisen die Hohlnadeln lokale Durchbrüche oder Öffnungen in der Wandung auf, durch die eine einzubringende Flüssigkeit austreten kann.

Weitere Hautstechwerkzeuge sind aus den folgenden Dokumenten bekannt: EP 1 576 982 A1, US 2012/0295549 A1 und US 2007/038181 A1. Die Nadeln der bekannten Stechwerkzeuge können als Vollmaterialnadeln ausgebildet sein.

### Zusammenfassung

Aufgabe der Erfindung ist es, ein Hautstechwerkzeug sowie ein Handgerät zum lokalen Aufstechen einer menschlichen oder einer tierischen Haut anzugeben, mit denen ein verbessertes lokales Aufstechen der Haut ermöglicht ist, um zum Beispiel den Substanzeintrag zu optimieren.

Zur Lösung der Aufgabe ist ein Hautstechwerkzeug zum lokalen Aufstechen einer menschlichen oder einer tierischen Haut nach dem unabhängigen Anspruch 1 geschaffen. Anspruch 14 betrifft ein Handgerät zum lokalen Aufstechen einer menschlichen oder einer tierischen Haut. Ausgestaltungen sind Gegenstand von abhängigen Ansprüchen.

Nach einem Aspekt ist ein Hautstechwerkzeug zum lokalen Aufstechen einer menschlichen oder einer tierischen Haut geschaffen, welches eine Stechnadel aufweist, bei der ein Nadelkörper einen proximalen Nadelabschnitt und einen distalen Nadelabschnitt aufweist, in welchem eine Stechspitze angeordnet ist. Der Nadelkörper weist einen bei Druckbeanspruchung gegen die Stechspitze federnden Nadelabschnitt auf. Die Nadel ist als Vollmaterialnadel ausgeführt.

Nach einem weiteren Aspekt ist ein Handgerät zum wiederholten lokalen Aufstechen einer menschlichen oder einer tierischen Haut geschaffen, bei dem das Hautstechwerkzeug an einem Nadelmodul angeordnet ist, welches an ein Antriebsmodul funktionell koppelt, mit dem repetierend eine Antriebskraft zum Aus- und Einfahren zumindest der Stechspitze des Hautstechwerkzeugs bereitgestellt ist.

Wird das Hautstechwerkzeug mit seiner Stechspitze gegen eine Fläche gedrückt, welche dem Drücken einen Widerstand entgegenstellt, gibt die Stechnadel federnd nach. Hierdurch kann der distale Nadelabschnitt in einer Ausgestaltung in Bezug auf die Längsrichtung des Nadelkörpers eine Kipp-, Klapp- oder Schwenkbewegung ausführen.

Der federnde Nadelabschnitt kann an dem Nadelkörper zwischen dem proximalen und dem distalen Nadelabschnitt angeordnet sein.

Der federnde Nadelabschnitt ist an dem Nadelkörper im Bereich eines gewinkelten Nadelabschnitts angeordnet, in welcher benachbarten Nadelabschnitte miteinander einen Winkel von weniger als 180° einnehmen. Die zu dem federnden Nadelabschnitt benachbarten Nadelabschnitte können hinsichtlich ihrer Formgestaltung gleich oder verschieden sein. Beispielsweise können die benachbarten Nadelabschnitte als gerade oder gekrümmte Nadelabschnitte ausgebildet sein. In einer Ausführung kann die elastische Federbewegung dazu führen, dass die benachbarten Nadelabschnitte aufeinander zu bewegt werden.

Der federnde Nadelabschnitt kann an dem Nadelkörper im Bereich eines gekrümmten Nadelabschnitts angeordnet sein. Auch in Verbindung mit einem gekrümmten federnden Nadelabschnitt können die hierzu benachbarten Nadelabschnitte gerade oder gekrümmt sein, wobei die benachbarten Nadelabschnitte die gleiche Form oder verschiedene Formen aufweisen können.

Ein weiterer federnder Nadelabschnitt kann an dem Nadelkörper im Bereich eines Schlaufen-Nadelabschnitts angeordnet sein. Es kann eine geschlossene Schlaufe gebildet sein. Der Schlaufen-Nadelabschnitt kann eine Kreisform aufweisen.

Der distale Nadelabschnitt kann zur Längsrichtung des Nadelkörpers schräggestellt sein. Die Längsrichtung des Nadelkörpers kann bei dieser oder anderen Ausführungsformen im Fall der Anwendung mit einer Stech- oder Applikationsrichtung zusammenfallen. Der distale Nadelabschnitt kann als gerader Nadelabschnitt ausgeführt sein, insbesondere auch von der Stechspitze ausgehend. Alternativ kann der distale Nadelabschnitt eine gekrümmte Form aufweisen, beispielsweise auch von der Stechspitze ausgehend.

Ein an den distalen Nadelabschnitt angrenzender Nadelabschnitt kann zur Längsrichtung des Nadelkörpers schräggestellt sein. Ist auch der distale Nadelabschnitt zur Längsrichtung des Nadelkörpers schräggestellt, kann die Schrägstellung des hieran angrenzenden Nadelabschnitts mit einem entgegengesetzten Winkel ausgebildet sein, wobei die Schrägstellungen in Bezug auf die Längsrichtung des Nadelkörpers mit gleichem Winkel oder unterschiedlichen Winkeln ausgebildet sein kann. In einer Ausführung können der distale Nadelabschnitt und der hieran angrenzende Nadelabschnitt einer liegenden V-Stellung entsprechend angeordnet sein, wobei in einer Ausgestaltung der federnde Nadelabschnitt zwischen den benachbarten Nadelabschnitten gebildet sein kann.

Zu dem federnden Nadelabschnitt können benachbarte Nadelabschnitte eingerichtet sein, beim Federn eine Scherenschenkel-Bewegung auszuführen.

Der federnden Nadelabschnitt kann in Bezug auf einen hierzu benachbarten Nadelabschnitt mit einer Materialverdünnung und / oder-verdickung gebildet sein. Die Verdünnung und / oder die Verdickung können mit Hilfe einer oberflächenseitigen Profilierung des Nadelkörpers im Bereich des federnden Nadelabschnitts gebildet sein.

Der Nadelkörper kann einen weiteren bei Druckbeanspruchung gegen die Stechspitze federnden Nadelabschnitt aufweisen. Der oder die federnden Nadelabschnitte können eingerichtet sein, als Reaktion auf eine im Wesentlichen parallel zur Längsrichtung des Nadelkörpers gerichtete Druckkraft zu federn. Die federnde Nadelabschnitte können in gleicher oder unterschiedlicher Ausprägung gebildet sein, beispielsweise hinsichtlich einer Federkonstante und / oder bezüglich der vorgesehenen Materialform, zum Beispiel der Querschnittsform.

Eine weitere Stechnadel kann mit der Stechnadel eine Stechnadelgruppe bilden. Die Stechnadeln der Stechnadelgruppe können gleich oder verschieden ausgebildet sein.

Die jeweiligen federnden Nadelabschnitte der Stechnadel und der weiteren Stechnadel können nebeneinanderliegend angeordnet sein.

Die jeweiligen federnden Nadelabschnitte der Stechnadel und der weiteren Stechnadel können in Längsrichtung der Stechnadelgruppe versetzt zueinander angeordnet sein.

Ein Hautstechwerkzeug kann in einer Ebene nebeneinanderliegend angeordnet sein. Alternativ können die mehreren Stechnadeln der Stechnadelgruppe als nicht flaches Bündel von Nadeln ausgebildet sein.

Alternativ zum Handgerät zum wiederholten Aufstechen einer menschlichen oder einer tierischen Haut kann ein Handgerät mit dem Hautstechwerkzeug als Einzelstechhandgerät vorgesehen sein. Ein solches Handgerät kann ohne das Antriebsmodul ausgeführt sein, mit dem automatisch repetierend die Antriebskraft bereitgestellt wird, zum Beispiel mit einer Wiederholfrequenz von wenigstens 50 Hz. Derartige Handgeräte sind als solche beispielweise in den folgenden Dokumenten offenbart: EP 1 872 823 A1, EP 1 958 659 A1, EP 2 149 388 A1 und EP 2 682 146 A1.

### Beschreibung von Ausführungsbeispielen

Im Folgenden werden weitere Ausführungsbeispiele unter Bezugnahme auf Figuren einer Zeichnung erläutert. Hierbei zeigen:
- Fig. 1: schematische Darstellung einer Stechnadel mit einem ausgeprägten gekrümmten Nadelabschnitt,
- Fig. 2: schematische Darstellungen von Stechnadeln mit einem abgewinkelten Nadelabschnitt,
- Fig. 3: schematische Darstellungen von Stechnadeln mit einem Schlaufen-Nadelabschnitt,
- Fig. 4: eine schematische Darstellung zur Wirkungsweise einer Ausführungsform einer Stechnadel,
- Fig. 5: eine schematische Darstellung zur Wirkungsweise einer weiteren Ausführungsform einer Stechnadel,
- Fig. 6: schematische Darstellungen von Stechnadelgruppen mit Stechnadeln mit gekrümmtem Nadelabschnitt,
- Fig. 7: schematische Darstellungen von Stechnadelgruppen mit Stechnadeln mit gewinkeltem Nadelabschnitt,
- Fig. 8: eine perspektivische Darstellung einer Stechnadelgruppe,
- Fig. 9: eine perspektivische Darstellung einer anderen Stechnadelgruppe, und
- Fig. 10: eine schematische Darstellung eines Handgeräts zum wiederholten Aufstechen einer Haut.

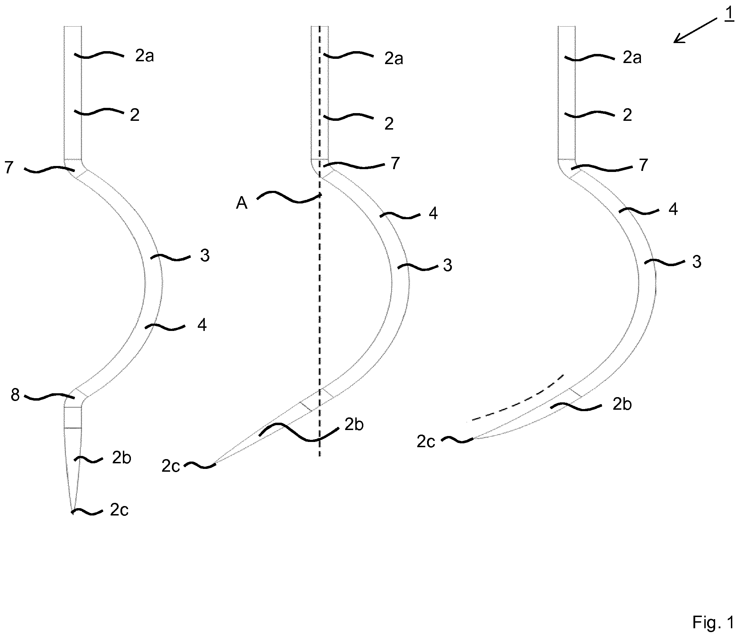

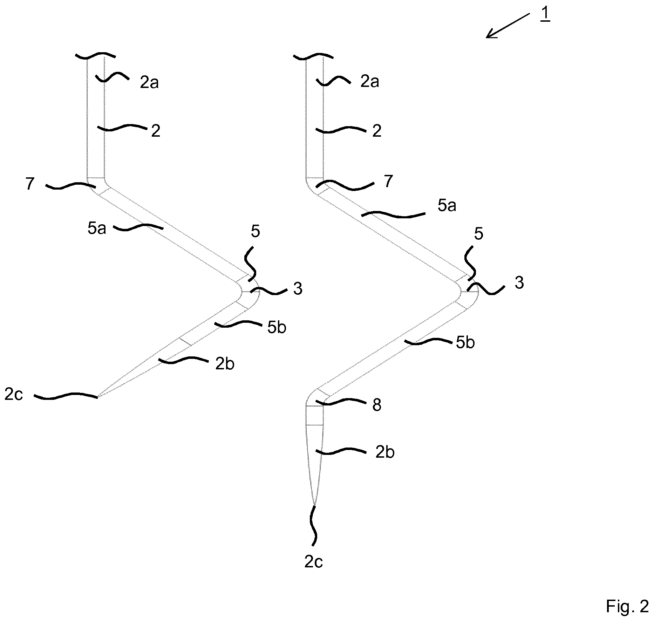

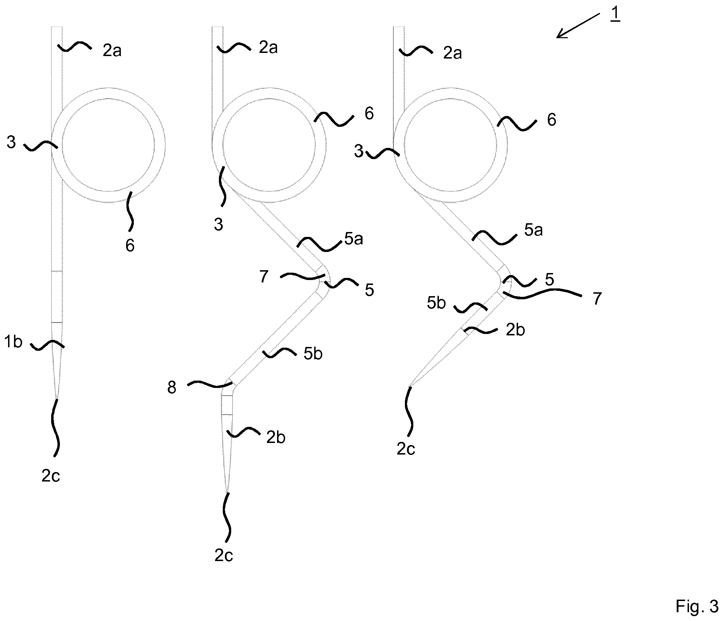

Die Fig. 1 bis 3 zeigen Ausführungsformen für eine einzelne Stechnadel 1, bei der am Nadelkörper 2 ein federnder Nadelabschnitt 3 vorgesehen ist. Während Fig. 1 Ausführungsformen zeigt, bei denen der Nadelkörper 2 einen gekrümmten Nadelabschnitt 4 aufweist, sind in Fig. 2 Ausführungsformen gezeigt, bei denen an einen abgewinkelten Bereich 5 benachbarte gerade Nadelabschnitte 5a, 5b anschließen. Fig. 3 zeigt Ausführungsformen, bei denen am Nadelkörper 2 ein Schlaufen-Nadelabschnitt 6 einen federnden Nadelabschnitt bildet. Alternativ zur gezeigten Ausgestaltung kann die Lage vom Schlaufen-Nadelabschnitt 6 mit abgewinkeltem Bereich 5 vertauscht sein.

Die einzelnen Stechnadeln 1 verfügen am Nadelkörper 2 über einen proximalen und einen distalen Nadelabschnitt 2a, 2b, wobei am distalen Nadelabschnitt eine Stechspitze 2c angeordnet ist. Der proximale Nadelabschnitt 2a kann als ein Nadelschaft gebildet sein, welcher zum Befestigen der Stechnadel 1 an einem Handgerät zum lokalen Aufstechen einer Haut nutzbar ist.

In Fig. 1 ist mittels einer gestrichelten Linie A schematisch eine Längsrichtung des Nadelkörpers 2 gezeigt.

Bei den verschiedenen Ausführungsformen kann zumindest ein weiterer federnder Nadelabschnitt 7, 8 gebildet sein. Alternativ kann die Stechnadel 1 in diesem Nadelabschnitt auch im Wesentlichen nicht federnd ausgebildet sein, so dass es bei Druckbeaufschlagung der Stechspitze 1c zum Federn nur im Bereich des federnden Nadelabschnitts 3 kommt.

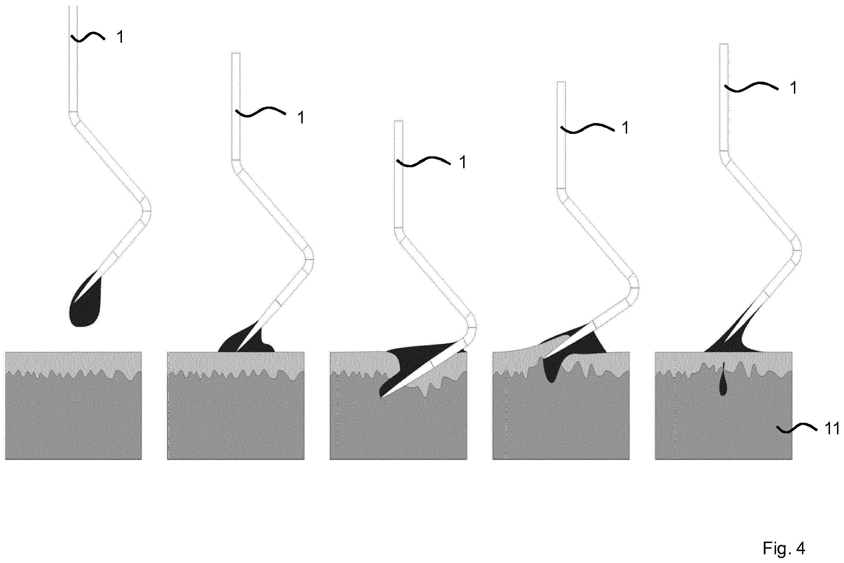

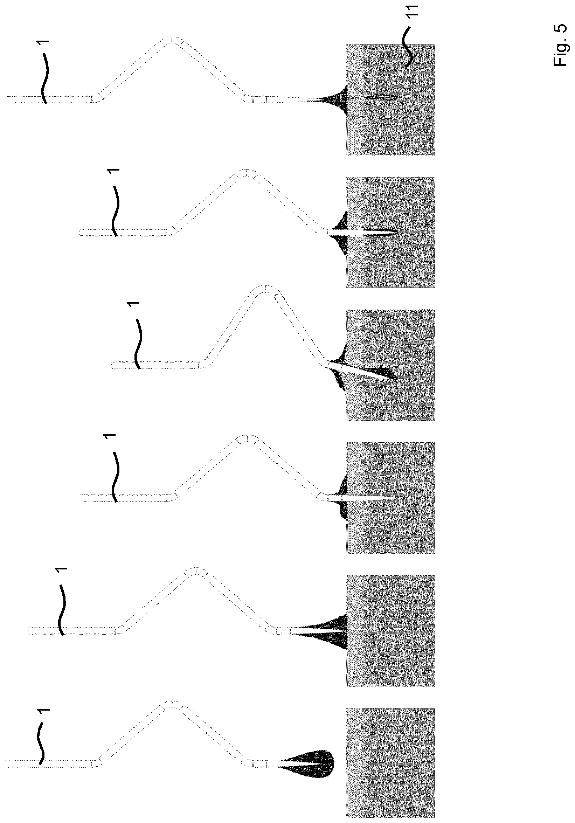

Das Vorsehen des oder der federnden Nadelabschnitt 3, 7, 8 führt im Einsatz der Stechnadel 1 zum Kippen oder Schwenken zumindest des distalen Nadelabschnitts 2b, wenn die Stechspitze 1c mit Druck beaufschlagt wird beim Aufstechen der Haut. Dieses ist in den Fig. 4 und 5 schematisch gezeigt. Auf diese Weise kann der Substanzeintrag in eine Haut 11 verbessert werden, für die in den Fig. 4 und 5 ein Abschnitt im Querschnitt gezeigt ist.

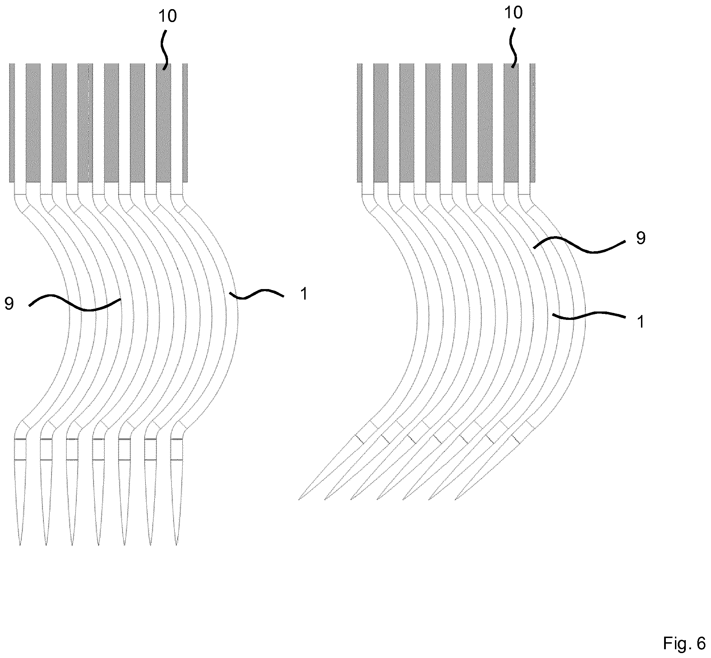

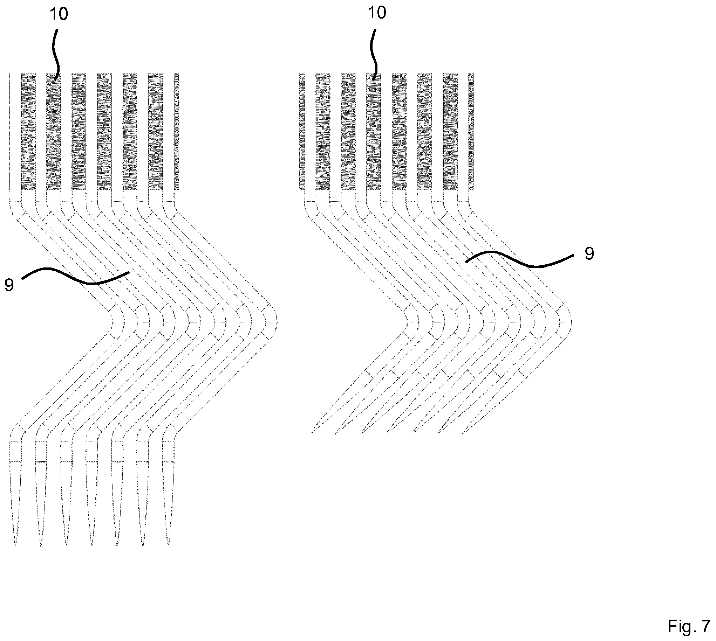

Die Fig. 6 und 7 zeigen schematische Darstellungen von Stechnadelgruppen 9, bei denen mehrere der in den Fig. 1 bis 3 gezeigten Stechnadeln 1, die an einer Nadelaufnahme 10 angeordnet sind, ein Hautstechwerkzeug bilden.

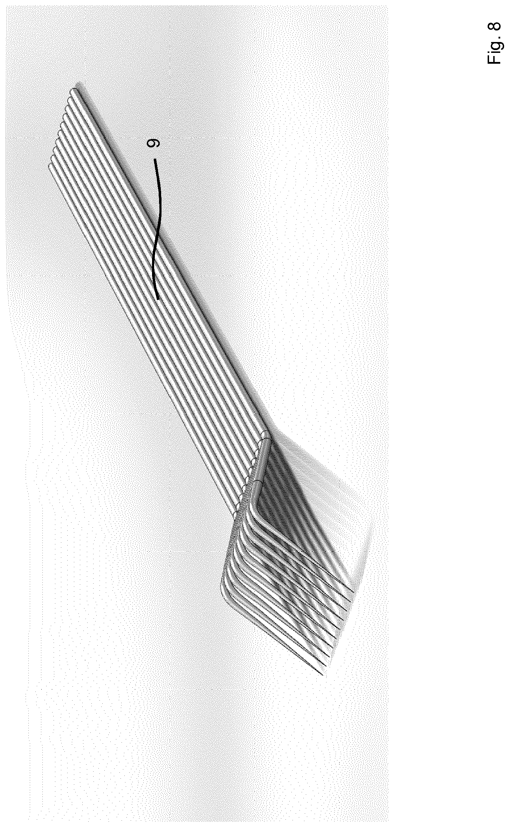

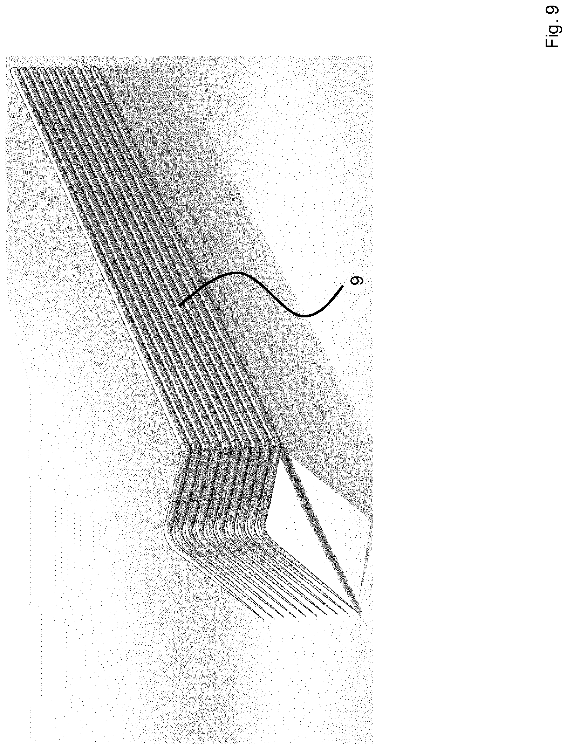

Fig. 8 und 9 zeigen jeweils eine perspektivische Darstellung einer Stechnadelgruppe 9, bei der proximale Nadelabschnitte 2a mehrerer Stechnadeln 1 in einer Ebene nebeneinander angeordnet sind.

Die gezeigten Ausführungsformen der Stechnadel 1 sind als Vollmaterialnadel ausgeführt.

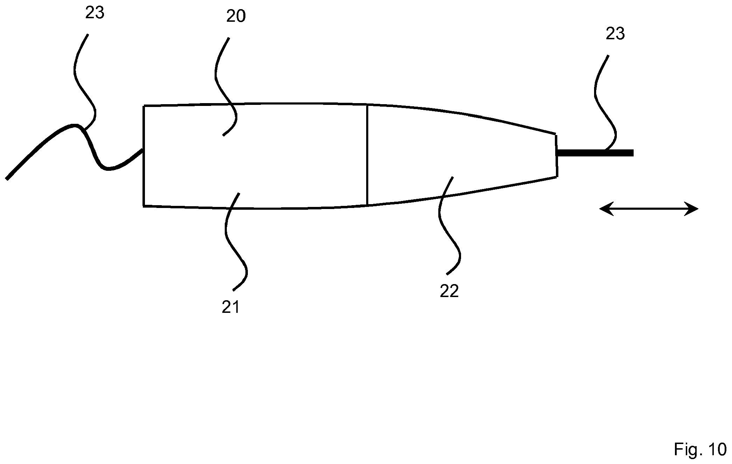

Fig. 10 zeigt eine schematische Darstellung eines Handgeräts 20 zum wiederholten Aufstechen einer Haut mit einem Antriebsmodul 21, an welches ein Nadelmodul 22 funktionell koppelt. Am Nadelmodul 22 ist ein Hautstechwerkzeug 23 in einer der hier beschriebenen Ausführungen angeordnet. Das Antriebsmodul ist über ein Kabel 24 an eine elektrische Spannungsversorgung anschließbar, zum Beispiel über ein Steuergerät (nicht dargestellt). Auch ein kabelloser Akkubetrieb kann vorgesehen sein.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Hautstechwerkzeug zum lokalen Aufstechen einer menschlichen oder einer tierischen Haut, mit einer Stechnadel, bei der ein Nadelkörper (2) einen proximalen Nadelabschnitt (2a) und einen distalen Nadelabschnitt (2b) aufweist, in welchem eine Stechspitze (2c) angeordnet ist, wobei der Nadelkörper (2) einen bei Druckbeanspruchung gegen die Stechspitze federnden Nadelabschnitt aufweist, wobei der federnde Nadelabschnitt an dem Nadelkörper (2) im Bereich eines gewinkelten Nadelabschnitts (3; 5; 7; 8) angeordnet ist, in welchem benachbarte Nadelabschnitte miteinander einen Winkel von weniger als 180° einnehmen, **dadurch gekennzeichnet dass** die Stechnadel als Vollmaterialnadel ausgeführt ist.

2. Hautstechwerkzeug nach Anspruch 1, **dadurch gekennzeichnet, dass** der federnde Nadelabschnitt an dem Nadelkörper (2) zwischen dem proximalen und dem distalen Nadelabschnitt (2a, 2b) angeordnet ist.

3. Hautstechwerkzeug nach Anspruch 1 oder 2, **dadurch gekennzeichnet, dass** der distale Nadelabschnitt (2b) zur Längsrichtung des Nadelkörpers (2) schräggestellt ist.

4. Hautstechwerkzeug nach mindestens einem der vorangehenden Ansprüche, **dadurch gekennzeichnet, dass** ein an den distalen Nadelabschnitt (2b) angrenzender Nadelabschnitt zur Längsrichtung des Nadelkörpers (2) schräggestellt ist.

5. Hautstechwerkzeug nach mindestens einem der vorangehenden Ansprüche, **dadurch gekennzeichnet, dass** zu dem federnden Nadelabschnitt benachbarte Nadelabschnitte eingerichtet sind, beim Federn einen Scherenschenkel-Bewegung auszuführen.

6. Hautstechwerkzeug nach mindestens einem der vorangehenden Ansprüche, **dadurch gekennzeichnet, dass** der federnden Nadelabschnitt in Bezug auf einen hierzu benachbarten Nadelabschnitt mit einer Materialverdünnung und / oder -verdickung gebildet ist.

7. Hautstechwerkzeug nach mindestens einem der vorangehenden Ansprüche, **dadurch gekennzeichnet, dass** der Nadelkörper (2) einen weiteren bei Druckbeanspruchung gegen die Stechspitze federnden Nadelabschnitt aufweist.

8. Hautstechwerkzeug nach Anspruch 7, **dadurch gekennzeichnet, dass** der weitere federnde Nadelabschnitt an dem Nadelkörper (2) im Bereich eines gekrümmten Nadelabschnitts (4) angeordnet ist.

9. Hautstechwerkzeug nach Anspruch 7, **dadurch gekennzeichnet, dass** der weitere federnde Nadelabschnitt an dem Nadelkörper (2) im Bereich eines Schlaufen-Nadelabschnitts (6) angeordnet ist.

10. Hautstechwerkzeug nach mindestens einem der vorangehenden Ansprüche, **gekennzeichnet durch** mindestens eine weitere Stechnadel, die mit der Stechnadel eine Stechnadelgruppe (9) bildet.

11. Hautstechwerkzeug nach Anspruch 10, **dadurch gekennzeichnet, dass** die jeweiligen federnden Nadelabschnitte der Stechnadel und der weiteren Stechnadel nebeneinanderliegend angeordnet sind.

12. Hautstechwerkzeug nach Anspruch 10, **dadurch gekennzeichnet, dass** die jeweiligen federnden Nadelabschnitte der Stechnadel und der weiteren Stechnadel in Längsrichtung der Stechnadelgruppe (9) versetzt zueinander angeordnet sind.

13. Hautstechwerkzeug nach mindestens einem der Ansprüche 10 bis 12, **dadurch gekennzeichnet, dass** die Stechnadel und die weitere Stechnadel in einer Ebene nebeneinanderliegend angeordnet sind.

14. Handgerät zum wiederholten lokalen Aufstechen einer menschlichen oder einer tierischen Haut, bei ein Hautstechwerkzeug nach mindestens einem der vorangehenden Ansprüche an einem Nadelmodul angeordnet ist, welches an ein Antriebsmodul funktionell koppelt, mit dem repetierend eine Antriebskraft zum Aus- und Einfahren zumindest der Stechspitze des Hautstechwerkzeugs bereitgestellt ist.

## Claims

1. Skin piercing tool for locally piercing a human or animal skin, comprising a piercing needle in which a needle body (2) has a proximal needle section (2a) and a distal needle section (2b) in which a piercing tip (2c) is arranged, wherein the needle body (2) has a needle section which is biased resiliently against the piercing tip in response to compressive load, wherein the resilient needle section is arranged on the needle body (2) in the area of an angled needle section (3; 5; 7; 8) in which adjacent needle sections form an angle of less than 180° with one another, **characterized in that** the piercing needle is embodied as a solid material needle.

2. The skin piercing tool according to Claim 1, **characterized in that** the resilient needle section is arranged on the needle body (2) between the proximal and the distal needle sections (2a; 2b).

3. The skin piercing tool according to Claim 1 or 2, **characterized in that** the distal needle section (2b) is positioned at an incline to the longitudinal direction of the needle body (2).

4. The skin piercing tool according to at least one of the preceding claims, **characterized in that** a needle section adjacent to the distal needle section (2b) is positioned at an incline to the longitudinal direction of the needle body (2).

5. The skin piercing tool according to at least one of the preceding claims, **characterized in that** needle sections adjacent to the resilient needle section are configured to perform a scissor-leg movement upon biasing.

6. The skin piercing tool according to at least one of the preceding claims, **characterized in that** the resilient needle section is constructed with a thinning and/or thickening of the material relative to a needle section adjacent thereto.

7. The skin piercing tool according to at least one of the preceding claims, **characterized in that** the needle body (2) has a further needle section which is biased against the piercing tip in response to compressive load.

8. The skin piercing tool according to Claim 7, **characterized in that** the further resilient needle section is arranged on the needle body (2) in the area of a curved needle section (4).

9. The skin piercing tool according to Claim 7, **characterized in that** the further resilient needle section is arranged on the needle body (2) in the area of a loop pin section (6).

10. The skin piercing tool according to at least one of the preceding claims, **characterized by** at least one further piercing needle, which forms a piercing needle group (9) with the piercing needle

11. The skin piercing tool according to Claim 10, **characterized in that** the respective resilient needle sections of the piercing needle and of the further piercing needle are arranged so as to be located beside one another.

12. The skin piercing tool according to Claim 10, **characterized in that** the respective resilient needle sections of the piercing needle and of the further piercing needle are arranged so as to be offset relative to one another in the longitudinal direction of the piercing needle group (9).

13. The skin piercing tool according to at least one of Claims 10 to 12, **characterized in that** the piercing needle and the further piercing needle are arranged so as to be located beside one another in one plane.

14. A handheld device for repeated local piercing of a human or animal skin, wherein a skin piercing tool according to at least one of the preceding claims is arranged on a needle module, which functionally couples with a drive module, by means of which a drive force for extending and retracting at least the piercing tip of the skin piercing tool is provided repetitively.

## Revendications

1. Outil de piquage de la peau pour le piquage local d'une peau humaine ou animale, comportant une aiguille de piquage dans laquelle un corps d'aiguille (2) présente une section d'aiguille proximale (2a) et une section d'aiguille distale (2b) dans laquelle une pointe de piquage (2c) est disposée, le corps d'aiguille (2) présentant une section d'aiguille élastique en cas de sollicitation par pression contre la pointe de piquage, la section d'aiguille élastique étant disposée au niveau du corps d'aiguille (2) au niveau d'une section d'aiguille coudée (3 ; 5 ; 7 ; 8) dans laquelle des sections d'aiguille voisines adoptent les unes par rapport aux autres un angle de moins de 180°, **caractérisé en ce que** l'aiguille de piquage est réalisée sous forme d'une aiguille en matériau plein.

2. Outil de piquage de la peau selon la revendication 1, **caractérisé en ce que** la section d'aiguille élastique est disposée au niveau du corps d'aiguille (2) entre les sections d'aiguille proximale et distale (2a, 2b).

3. Outil de piquage de la peau selon la revendication 1 ou 2, **caractérisé en ce que** la section d'aiguille distale (2b) est positionnée obliquement par rapport au sens longitudinal du corps d'aiguille (2).

4. Outil de piquage de la peau selon au moins une des revendications précédentes, **caractérisé en ce qu'**une section d'aiguille adjacente à la section d'aiguille distale (2b) est positionnée obliquement par rapport au sens longitudinal du corps d'aiguille (2).

5. Outil de piquage de la peau selon au moins une des revendications précédentes, **caractérisé en ce que** les sections d'aiguille voisines de la section d'aiguille élastique sont conçues pour exécuter lors du mouvement de ressort un mouvement de branches de ciseaux.

6. Outil de piquage de la peau selon au moins une des revendications précédentes, **caractérisé en ce que** la section d'aiguille élastique est constituée, par rapport à une section d'aiguille voisine, avec un amincissement et/ou un épaississement de matériau.

7. Outil de piquage de la peau selon au moins une des revendications précédentes, **caractérisé en ce que** le corps d'aiguille (2) présente une autre section d'aiguille élastique en cas de sollicitation de pression contre la pointe de piquage.

8. Outil de piquage de la peau selon la revendication 7, **caractérisé en ce que** l'autre section d'aiguille élastique est disposée au niveau du corps d'aiguille (2) au niveau d'une section d'aiguille courbée (4).

9. Outil de piquage de la peau selon la revendication 7, caractérisé l'autre section d'aiguille élastique est disposée au niveau du corps d'aiguille (2) au niveau d'une section d'aiguille en boucle (6).

10. Outil de piquage de la peau selon au moins une des revendications précédentes, **caractérisé par** au moins une autre aiguille de piquage qui constitue avec l'aiguille de piquage un groupe d'aiguilles de piquage (9).

11. Outil de piquage de la peau selon la revendication 10, **caractérisé en ce que** les sections d'aiguille élastiques respectives de l'aiguille de piquage et de l'autre aiguille de piquage sont disposées les unes près des autres.

12. Outil de piquage de la peau selon la revendication 10, **caractérisé en ce que** les sections d'aiguille élastiques respectives de l'aiguille de piquage et de l'autre aiguille de piquage sont disposées décalées les unes des autres dans le sens longitudinal du groupe d'aiguilles de piquage (9).

13. Outil de piquage de la peau selon au moins une des revendications 10 à 12, **caractérisé en ce que** l'aiguille de piquage et l'autre aiguille de piquage sont disposées les unes près des autres dans un plan.

14. Appareil manuel pour le piquage local répété d'une peau humaine ou animale, dans lequel est disposé dans un module d'aiguille un outil de piquage de la peau selon une des revendications précédentes qui se couple fonctionnellement à un module d'entraînement grâce auquel une force d'entraînement permettant de rentrer et sortir au moins la pointe de piquage de l'outil de piquage de la peau est mise à disposition à répétition.
